# FASCICULE DE BREVET EUROPEEN

(11) **EP 0 577 463 B1**
(45) Date de publication et mention de la délivrance du brevet: **31.10.2001**
(21) Numéro de dépôt: 93401576.9
(22) Date de dépôt: 21.06.1993
(51) Int. Cl.: C12P 17/06, A23L 1/23, C07D 309/30

(54) **Procédé pour la préparation de delta lactones saturées par biohydrogénation des composés insaturés naturels correspondants à l'aide de microorganismes**
Verfahren zur Herstellung von gesättigten Delta-Laktonen durch Biohydrierung von den übereinstimmenden ungesättigten Verbindungen mit Mikroorganismen
Process for the preparation of saturated delta lactones by biohydrogenation of the corresponding insaturated compounds by microorganisms

(30) Priorité: 22.06.1992 IT TO920532
(43) Date de publication de la demande: 05.01.1994
(73) Titulaire: San Giorgio Flavors, 10147 Torino (IT)
(72) Inventeur: Cardillo, Rosanna, I-20133 Milano (IT); Fuganti, Claudio, I-20129 Milano (IT); Barbeni, Massimo, I-10141 Torino (IT); Allegrone, Gianna, I-10141 Torino (IT)
(74) Mandataire: Warcoin, Jacques

(56) Documents cités:
- EP-A- 0 412 880
- EP-A- 0 425 001

## Description

La présente invention a pour objet un procédé pour la préparation de delta lactones saturées et en particulier de delta décanolide et/ou de delta dodécanolide par biohydrogénation des composés insaturés naturels correspondants, à l'aide de microorganismes.

La préférence des consommateurs pour l'emploi d'agents aromatisants naturels par rapport aux synthétiques a entraîné la recherche de méthodologies naturelles permettant l'accès à des quantités significatives de produits aromatisants non accessibles à bon marché par extraction à partir de sources naturelles.

Celles-ci comprennent la conversion enzymatique de substances naturelles accessibles à bon marché en celles désirées, présentes en traces dans la nature.

Une telle procédure, appelée bioconversion, a été particulièrement étudiée pour la production de gamma et delta lactones contenant de 6 à 12 atomes de carbone . Sont efficaces, en particulier, les procédés de bêta oxydation microbienne des dérivés hydroxylés d'acides gras naturels. De cette façon, aussi bien le gamma décanolide que le delta décanolide sont isolables à partir de processus de fermentation dans lesquels on administre, à un grand nombre de microorganismes, des acides gras insaturés C-18 contenant une fonction hydroxyle en positions 12 et 13, respectivement; toutefois, alors que le premier précurseur comme l'acide ricinoléique est abondant dans la nature, le second, l'acide coriolique, est d'un accès plus difficile.

La demande de brevet EP-A-O 412 880 décrit un procédé pour la production de gamma ou delta lactones saturées ou insaturées à partir de substrats contenant des hydroxydes ou hydroperoxydes d'acide linoléique, linolénique, ou oléique en employant des microorganismes capables d'effectuer une bêta oxydation des hydroxydes et hydroperoxydes susmentionnés.

En particulier, la demande de brevet susmentionnée décrit la production de delta décanolide en employant de l'acide coriolique produit sous la forme (S) par la lipooxygénation de l'acide linoléique suivie de la réduction de l'hydroperoxyde intermédiaire, ou par photo ou autooxydation du même acide ou de ses dérivés, suivie d'une réduction.

La demande de brevet canadienne 2,025,678 décrit une méthode alternative pour la production de gamma décanolide et de gamma dodécanolide par réduction bio-catalytique des lactones insaturées correspondantes en employant des levures ou des champignons.

En particulier, cette demande de brevet décrit la capacité de la levure de panification (Saccharomyces cerevisiae) et d'une série de champignons tels que Polyporus durus, Ischnoderma benzoinum, Bjerkandera Adusta, Poria xantha ou Pleurotus ostreatus de transformer le delta décen-2-olide en produit saturé correspondant par simple contact de la lactone insaturée de départ avec les biomasses indiquées ci-dessus, en présence d'un sucre utile pour le recyclage du cofacteur qui en s'oxydant, fournit l'hydrogène nécessaire à la réduction de la double liaison.

La particularité de ce processus tient au fait qu'un produit naturel est utilisé comme substrat, extrait par exemple de l'arbre de Massoia (Cryptocaria massoia).

Dans l'extrait naturel, aux côtés de la lactone insaturée C-10, est également présent l'homologue insaturé C-12, qui dans les processus indiqués ci-dessus subit une hydrogénation pour devenir la lactone saturée C-12 correspondante.

Le procédé décrit ci-dessus n'est toutefois pas pleinement satisfaisant. Quand on utilise la levure de panification, le rapport entre levure et lactone insaturée est très élevé; en particulier on indique l'utilisation de 9 g/litre de levure (poids sec) pour 10 mg de lactone insaturée. Alternativement, quand on effectue des additions successives de substrat, pour la même quantité de levure, des quantités modestes de produit saturé sont isolées. Etant donné le coût élevé du substrat naturel, ce fait constitue un inconvénient du point de vue économique. Il faut convenir que la grande quantité de biomasse rend difficile l'isolement du produit et/ou que celui-ci peut être biodégradé par la levure elle-même.

Les rapports biomasse/substrat insaturé sont plus favorables quand des champignons sont utilisés comme biocatalyseurs de réduction. On décrit des concentrations de produit saturé jusqu'à environ 600 mg/litre, mais par contre, les temps d'incubation sont beaucoup plus longs, jusqu'à 7 jours, et les temps de croissance du mycélium atteignent même quelques semaines.

Le but de la présente invention est d'améliorer le procédé décrit dans la demande de brevet susmentionnée, en effectuant une sélection de microorganismes qui permettent de travailler avec de faibles quantités de biomasse, des temps d'incubation brefs et des concentrations de substrat élevées.

Dans ce but, un sujet de la présente invention est un procédé pour la production de delta décanolide et/ou de delta dodécanolide ou de leurs mélanges grâce à la biohydrogénation d'un substrat contenant les lactones insaturées correspondantes, delta décen-2-olide, delta dodécen-2-olide ou leurs mélanges; ce procédé est caractérisé par le fait que la biohydrogénation est effectuée grâce à l'emploi du microorganisme Saccharomyces delbrueckii, en particulier, Saccharomyces dolbrueckii CBS 1146, désigné également par Torulaspora delbrueckii (changement de genre lors de la révision de la classification des levures, Yeasts characteristics an identification, Barnett et al., 2^{e}ed, Cambridge University Press) .

On relève que les cultures en croissance des microorganismes susmentionnés sont capables de réduire la lactone insaturée et son homologue supérieur, soit à l'état pur soit tels que présents dans l'extralt de Massola, pour donner les composés saturés correspondants avec des rendements molaires de produit isolé qui atteignent 85 %, avec des concentrations en substrat qui atteignent 1,5 g/litre et des temps de contact entre 24 et 48 heures.

Typiquement, le micro-organisme est mis en croissance sur milieu solide MPGA (20, 5, 20, 15) à 27-30°C. Celui-ci est utilisé pour préparer le pré-inoculat en milieu liquide MPGB pendant 24 heures; 50ml de ce pré-inoculat sont utilisés pour ensemencer à 10% des matras de 300ml contenant 50ml de MPGB. Après 24-48 heures, on ajoute le delta decen-2-olide (Massoria lactone) pur ou sous forme d'extrait de Massoria ou un mélange de celui-ci avec son homologue supérieur delta dodecen-2-olide ou encore ce dernier à l'état pur à 1-2 g/litre. La culture est maintenue sous agitation à 30°C pendant 24-48 heures.

Au moment de l'addition du substrat, une quantité équimolaire de bêta cyclodextrine peut être ajoutée.

Après le temps indiqué, la culture est portée à pH 3 et les produits de transformation sont récupérés selon les techniques conventionnelles adaptées à ce but, par exemple, par extraction avec un solvant organique, de préférence le chlorure de méthylène ou l'acétate d'éthyle.

Le solvant est séché, par exemple au sulfate de sodium, évaporé et le résidu est distillé sous vide. La composition est déterminée par analyse GLC et, dans certains cas, l'analyse quantitative est effectuée en mesurant le rapport entre les produits de transformation et un standard interne ajouté au moment de l'extraction.

De cette façon, on observe que la biohydrogénation des lactones insaturées est complète, dans certains cas en 24 heures et dans d'autres cas, le phénomène se vérifie au bout de 24 heures supplémentaires de contact.

La quantité de biomasse produite est plutôt modeste et telle qu'elle ne crée aucun problème d'émulsion ou perte de produit durant la récupération.

### Exemple illustratif 1

Des cultures de Debaryomyces hansenii CBS 767, mises en croissance trois jours sur MPGA, sont utilisées pour inoculer 50 ml de MPGA, qui après 24 heures de croissance à 27-30°C sous agitation servent à inoculer 500 ml de MPGA. Après 48 heures de croissance sous agitation, à 10 matras de 300 ml contenant 50 ml de culture sont ajoutés 50 mg de Massoia lactone. On maintient sous agitation pendant 24 heures et après cet intervalle de temps, les cultures sont réunies, le pH est porté à 3, trois extractions avec 50 ml de chlorure de méthylène sont effectuées. La phase organique séchée est évaporée avec une colonne de rectification et le résidu est distillé sous vide. On obtient 420 mg de distillat contenant environ 90 % de delta décanolide dépourvu de produit de départ.

Quand on utilise, respectivement, 75 et 100 mg de substrat insaturé pour 50 ml de culture, après 24 heures, on observe à l'analyse GLC un rapport insaturé/saturé.

### Exemple illustratif 2

Des cultures de Pichia ohmeri CBS 5367 mises en croissance dans des conditions identiques à celles de l'exemple 1, sont traitées respectivement avec 0,75 et 1 g/litre de Massoia lactone. Après 24 heures d'incubation on a observé une réduction complète du composé insaturé et une récupération de 75 % du produit.

### Exemple illustratif 3

Des cultures de Hansenula anomala CBS 110, mises en croissance de la même façon, traitées avec 500 mg/litre d'essence de Massoia (Robertet) contenant la lactone insaturée C-10 et son homologue supérieur C-12, fournissent après 48 heures un mélange de produits contenant exclusivement les produits saturés delta décanolide et delta dodécanolide dans les mêmes rapports que ceux dans lesquels se trouvaient les composés insaturés correspondants.

### Exemple 1

Des cultures de Saccharomyces delbrueckii CBS 1146, mises en croissance comme dans l'exemple précédent, traitées avec 1 g/litre de Massoia lactone, fournissent après 24 heures d'incubation un mélange environ 1:1 de produits saturés et insaturés. En laissant encore 24 heures, une saturation complète est observée et le produit est isolé avec un rendement de 70 %.

### Exemple 2

Des cultures de Saccharomyces delbrueckii CBS 1146, mises en croissance comme dans l'exemple 1, sont traitées à la Massoia lactone (1 g/litre) et à la bêta cyclodextrine (8 g/litre). Après 24 heures, une saturation complète de la double liaison est observée, le produit saturé désiré est finalement isolé avec un rendement de 82 %.

### Exemple 3

On procède comme dans l'exemple 5, mais on ajoute 2 g/litre de Massoia lactone et 8 g/litre de bêta cyclodextrine. Après 24 heures d'incubation, on mesure le rapport entre lactone saturée et lactone insaturée, égal à 7:5. Après 48 heures, le composé insaturé a complètement disparu tandis qu'apparaissait le composé saturé désiré comme composant principal du mélange d'extraction.

## Revendications

1. Procédé pour la production de delta-décanolide et/ou de delta dodécanolide ou de leurs mélanges au moyen de la biohydrogénation d'un substrat contenant les lactones insaturées correspondantes, respectivement, delta décen-2-ôlide, delta dodécen-2-olide et leurs mélanges, **caractérisé en ce que** la biohydrogénation est effectuée grâce à l'emploi de Saccharomyces delbrueckii.

2. Procédé selon la revendication 1, dans lequel le substrat est un extrait de Massoia.

3. Procédé selon la revendication 1 ou 2, dans lequel une quantité équimolaire de bêta cyclodextrine est ajoutée au substrat.

## Patentansprüche

1. Verfahren zur Herstellung von Delta-Decanolid und/oder Delta-Dodecanolid oder ihrer Mischungen mittels Biohydrierung eines Substrats, welches die entsprechenden ungesättigten Laktone, beziehungsweise Delta-Decen-2-olid, Delta-Dodecen-2-olid und ihre Mischungen, enthält, **dadurch gekennzeichnet, dass** die Biohydrierung durch die Verwendung von Saccaromyces delbrueckii bewirkt wird.

2. Verfahren gemäß Anspruch 1, wobei das Substrat ein Massoia-Extrakt ist.

3. Verfahren gemäß Anspruch 1 oder 2, wobei dem Substrat eine äquimolare Menge an Beta-Cyclodextrin zugegeben wird.

## Claims

1. Process for the production of delta-decanolide and/or delta-dodecanolide or mixtures thereof, by means of the biohydrogenation of a substrate containing the corresponding unsaturated lactones, delta-decen-2-olide and delta-dodecen-2-olide, respectively, and mixtures thereof, **characterised in that** the biohydrogenation is performed by means of the use of Saccharomyces delbrueckii

2. Process according to Claim 1, in which the substrate is a Massoia extract.

3. Process according to Claim 1 or 2, in which an equimolar amount of beta-cyclodextrin is added to the substrate.
